# EUROPEAN PATENT APPLICATION

(11) **EP 1 433 487 A1**
(43) Date of publication of application: **30.06.2004**
(21) Application number: 02796177.0
(22) Date of filing: 21.08.2002
(51) Int. Cl.: A61L 27/00, A61P 19/00, C12N 5/06

(54) **METHOD OF BONE REGENERATION**

(30) Priority: 22.08.2001 JP 2001251365
(71) Applicant: National Institute of Advanced Industrial Science and Technology, Tokyo 100-8921 (JP); Hata, Jun-ichi, Tokyo 141-0031 (JP); Umezawa, Akihiro, Matsudo-shi, Chiba 270-0014 (JP); Tateishi, Tetsuya, Tsukuba-shi, Ibaraki 305-0042 (JP); Ushida, Takashi, Tsukuba-shi, Ibaraki 305-0821 (JP)
(72) Inventor: HATA, Jun-ichi, Shinagawa-ku, Tokyo 141-0031 (JP); UMEZAWA, Akihiro, Matsudo-shi, Chiba 270-0014 (JP); TATEISHI, Tetsuya, Tsukuba-shi, Ibaraki 305-0042 (JP); USHIDA, Takashi, Tsukuba-shi, Ibaraki 305-0821 (JP); CHEN, Guoping, Tsukuba-shi, Ibaraki 305-0821 (JP)
(74) Representative: HOFFMANN - EITLE
(86) International application number: PCT/JP2002/008420
(87) International publication number: WO 2003/018077

(57) **Abstract**

This invention aims at providing a method of rapidly generating bone tissue having the mechanical strength, shape and size suitable for use in implantation, to provide the obtained normal regenerated bone tissue, and to provide a bone treatment material utilizing the same. Bone tissue suitable for implantation, having a specific shape and size is generated/regenerated by the proliferation of mesenchymal stem cells having the multi-potentiality for differentiation or osteoblasts in a fibrous and/or porous material which can serve as a scaffold for these cells.

## Description

### Technical Field

This invention relates to a novel method of regenerating (or generating) bone, and to normal regenerated bone tissue obtained by the method. This invention also relates to a bone treatment material utilizing the obtained regenerated bone tissue.

### Background Art

In the field of regenerative medicine, it has been proposed to restore damaged or diseased tissues using a cell-mediated method. It is thought that particularly, this technique can be most applied to restoration of bone and cartilage tissues. As a cell source for restoration of tissue, although embryonic stem cells, fetal cells or adult cells such as marrow stromal cells can be used, all these cells have their benefits and drawbacks; and it is impossible to decide which cell is best in all cases.

In recent years, mesenchymal stem cells possessing the multi-potentiality for differentiation have been isolated from adult bone marrow stroma (mesenchyme). The mesenchymal stem cells play a useful role in regeneration of mesenchymal tissues such as bone, cartilage, muscle, ligament, tendon, fatandstroma. Moreover, these stem cells can be easily isolated from the bone marrow and the periosteum of a living body. They display a stable phenotype and cell morphology of a stem cell; and for example, mesenchymal stem cells derived from bone marrow have the character of amonolayer ininvitroculturing (Pittenger M.F. et al. , Science 284, 143-147, 1999). Mesenchymal stem cells can be made to differentiate into various types of cells such as osteoblasts, chondrocytes and myoblasts *in vitro* by cytokines or growth factors. In particular, it is known that the differentiation to osteoblasts can be induced by the action of a bone growth factor (for example, BMP-2 or BMP-7) and that when a collagen substrate is implanted to humans with BMP-2 or BMP-7, normal bone tissue will be generated.

For this reason, it has been proposed to use bone tissue generated from mesenchymal stem cells or osteoblasts, or a collagen substrate containing a bone growth factor, as an implant for the implantation (or transplantation) to a patient who has a bone disease such as osteogenesis imperfecta or osteoporosis. However, although the collagen substrate is a material with excellent physiological affinity, it is difficult to give the material a specific shape and it does not have the sufficient mechanical strength to serve as bone tissue. Likewise, it is difficult to generate the bone tissue the differentiation of which has been induced *in vitro* into a specific shape, and the bone tissue lacks the mechanical strength of bone tissue. Further, in order to generate bone tissue *in vitro* to a size which can be used as an implant, a very long culturing time is required. Thus, there is a need for a method of regenerating bone tissue in a short period of time for implantation which has the mechanical strength of bone tissue.

Accordingly, this invention provides a method of rapidly generating bone tissue having mechanical strength, shape and size which can be used for implantation. It is also an object of this invention to provide the normal regenerated bone tissue thus obtained, and a bone treatment material utilizing the same.

### Disclosure of the Invention

As a result of intensive research to solve the aforesaid problem, the present inventors discovered that mesenchymal stem cells or osteoblasts possessing the multi-potentiality for differentiation are allowed to proliferate in a fibrous and/or porous material which can serve as a scaffold for these cells and thereby bone tissue of a specific shape and size suitable for implantation can rapidly be generated or regenerated, and thus arrived at this invention.

This invention, therefore, provides a method of regenerating bone, comprising allowing mesenchymal stem cells to proliferate in a fibrous and/or porous material which can serve as a scaffold for the cells.

It is preferred that the aforesaid material has a mechanical strength which can withstand implantation.

Moreover, as the material serves as a scaffold for the proliferating cells and remains in the body for a long period of time after implantation, it is preferred that the material has the property of not affecting the body, i.e., it is physiologically inert, and has biodegradability and physiological affinity so that it decomposes and is absorbed over time. The fibrous and/or porous material in this invention, therefore,preferably comprises a combination of a material having physiological affinity and a biodegradable polymer having mechanical strength. This invention, therefore, relates to a method of regenerating bone comprising allowing mesenchymal stem cells or osteoblasts to proliferate in a fibrous and/or porous medium comprising a combination of a material having physiological affinity and a biodegradable polymer having mechanical strength, which can serve as a scaffold for the cells.

The fibrous and/or porous material in this invention can easily be formed in a desired shape and desired size, and the regenerated bone tissue having the shape and size required for implantation can be obtained by adding cells to the formed material, and allowing them to proliferate. Accordingly, this invention relates to a method of easily obtaining a regenerated bone tissue of shape and size required for implantation, to the normal regenerated bone tissue thus obtained, and to a bone treatment material utilizing the same.

### Brief Description of the Drawings

FIG. 1 is a graph showing the DNA content of KUSA/A1 cells in culture, where it is seen that the KUSA/A1 cells proliferate normally.
FIG. 2 is a graph showing the ALP activity of KUSA/A1 cells in culture, where it is seen that the KUSA/A1 cells have the character of osteoblasts.
FIG. 3 is a graph showing the Ca content of KUSA/A1 cells in culture, where it is seen that the KUSA/A1 cells have the character of osteoblasts.
FIG. 4 is a graph showing the osteocalcin production/secretion amount of KUSA/A1 cells in culture, where it is seen that the KUSA/A1 cells have the character of osteoblasts.
FIG. 5 are photographs obtained from an X-ray (A) and histological examinations (B) of a porous material comprising KUSA/A1 cells, collagen and PLGA sponge 6 weeks after the implantation to a mouse, where it is seen that the bone tissue has regenerated in the implant.
FIG. 6 are photographs obtained from an X-ray (A) and histological examinations (B) of a porous material comprising KUSA/A1 cells, collagen and PLGA sponge 11 weeks after the implantation to a mouse, where it is seen that the bone tissue has completely regenerated in the implant, and PLGA has been replaced by bone tissue.
FIG. 7 shows the implantation of only the porous material comprising collagen and PLGA sponge to a mouse, where it is seen that bone tissue has not regenerated at all.
FIG. 8 is an X-ray photograph (B) 4 weeks after implanting a cell-inoculated fibrous material comprising KUSA/A1 cells, collagen and PLGA sponge to a mouse (A) with a bone defect created in the skull.
FIG. 9 is an X-ray photograph (B) 4 weeks after implanting a cell-inoculated fibrous material comprising KUSA/A1 cells, collagen and PLGA sponge to a mouse (A) with a bone defect in the femur.

### Best Mode for Carrying Out the Invention

### Cells

In this invention, the mesenchymal stem cells are of bone marrow and/or periosteum origin, and are undifferentiated cells possessing the multi-potentiality capable of differentiating into mesenchymal tissues, such as fat tissue, cartilage tissue or bone tissue. Although the mesenchymal stem cells can be extracted from any bone marrow or periostea which have these cells, it is desirable to extract them from the sternum, humerus, femur, tibia and pelvis (ileum). In the case of mammals other than humans, mesenchymal stem cells can be extracted from the ileum or the tibia.

As the method for extracting these mesenchymal stem cells from bone marrow, a method known in the art, for example any extraction methods practiced in medical treatment, can be used. If these stem cells are extracted from the bone marrow of mammals other than humans, in the laboratory procedure, both ends of the bone (femur or tibia) are cut, the inside of the bone is washed with a medium suitable for culturing mesenchymal stem cells, and the mesenchymal stem cells may be collected from this medium wash. The actual extraction is performed as follows:
(1) Both ends of the femur or tibia of a rabbit are cut. Subsequently, the inner bone marrow is washed with a medium (for example, DMEM medium), and if desired, with a medium to which antibiotics (penicillin, streptomycin, etc.) and heparin have also been added.
(2) The washed-out medium is centrifuged, solid lumps are removed as precipitates, the cells in the supernatant are measured as mesenchymal stem cells, and diluted to a suitable concentration with a suitable medium.

To extract these stem cells from the periosteum, a method known in the art, for example, the method of M. Iwasaki et al (Endocrinology 132, 1603-1608 (1993); J Fang& B.K Hall, Developmental Biol. 180, 701-712 (1996); and S. Bahrami et al., The Anatomical Record 259, 124-130 (2000)) may be followed to obtain them.

Using such a method, the desired number of mesenchymal stem cells can be obtained. The isolated mesenchymal stem cells may be inoculated as they are and provided for culturing, but they may usually be used after culturing in a suitable medium. Moreover, these cells can also be cryopreserved.

To obtain osteoblasts from mesenchymal stem cells, the stem cells are treated with trypsin, and isolated by centrifugation. They are then cultured in a medium which contains a growth factor promoting osteogenesis, such as bone growth factors (BMP-2 and BMP-7). Methods and cultures suitable for inducing bone differentiation are disclosed in detail, for example, in M.F. Pittenger et al., Science 284, pp.143-147, 1999.

### Fibrous/porous material

The form of the fibrous and/or porous material in this invention is one that can form a three-dimensional structure, for example, a porous material (such as a sponge), a mesh (such as a textile) , cloth, unwoven cloth or cotton. The use of a porous material is preferred as mesenchymal stem cells or differentiated osteoblasts easily adhere to the material, and at the same time, penetrate the interior, therebybeing able to promote regeneration of bone tissue. Also, in order that mesenchymal stem cells and/or osteoblasts can proliferate on this material as a scaffold, it preferably contains, is bonded to or is covered by a material having physiological affinity so that it will be compatible with tissue after implantation.

In an embodiment of this invention, anymaterial having physiological affinity may be used, provided that it is a biopolymer, but it is preferably one or more biomaterials selected from the group consisting of collagen (collagen Types I, II, II, IV), polyethylene oxide, fibrin glue, gelatin, fibronectin, laminine, proteoglycans and glycosaminoglycan. Collagen Type I, II, III and/or IV is more preferable.

The biodegradable polymer having mechanical strength is preferably one or more biodegradable polymers selected from the group consisting of polyglycolic acid, polylactic acid, polylactic acid-polyglycolic acid copolymer, polymalic acid, chitin, chitosan, polyalginic acid, hyaluronic acid, cellulose, polyamino acid and ω-hydroxycarboxylic acid. It is more preferably polyglycolic acid, polylactic acid or polylactic acid-polyglycolic acid copolymer, andmostpreferablypolylactic acid-polyglycolic acid copolymer.

The larger the porosity and pore size of the biodegradable synthetic polymer structure having mechanical strength, the lower the mechanical strength becomes. However, as the connection between pores improves and cells can be easily inoculated, the number of inoculated cells in the structure can be increased, and regeneration of bone tissue becomes more efficient. Therefore, the porosity andpore size of the structure maybe adequately determined taking account of desired mechanical strength, elasticity or regeneration rate of bone tissue according to the site of the body to which it is implanted. The porosity is preferably 70% or more, and the pore size is 250 µm or more.

The porous structure (for example, sponge) of the biodegradable polymer having mechanical strength can be produced using the phase separation method, particulate leaching or the foaming method. The fibers (for example, mesh, woven cloth, unwoven cloth, cotton, etc.) of the biodegradable polymer can be produced using the melt spinning method or the elongation (stretching) method. In any of these methods, the biodegradable polymer is dissolved in a solvent, and its three-dimensional structure is then formed. The solvents used to dissolve the polymer include, but not limited to, chloroform, carbon tetrachloride, dioxane, trichloroacetic acid, dimethylformamide, methylene chloride, ethyl acetate, acetone, hexafluoroisopropanol, dimethylacetamide, hexafluoro-2-propanol, acetic acid, formic acid and water.

Additives, such as a surfactant, a stabilizer or an anti-oxidant, may also be added to the solution of biodegradable polymer as necessary.

The porous structure (for example, sponge) of the biodegradable polymer having mechanical strength can be produced using a particle or crystal such as a water-soluble saccharide, e.g. grape sugar, fruit sugar or sugar, a particle or crystal such as a water-soluble salt, e.g. sodium chloride, potassium chloride, sodium tartrate, sodium citrate, ammonium carbonate, sodium carbonate or sodium bicarbonate, or an inert gas, such as carbon dioxide or nitrogen.

The material having physiological affinity may be an aqueous solution, a solution of an organic solvent miscible with water such as alcohol, or a mixed solution thereof, but an aqueous solution is preferred. The aqueous solution of the material having physiological affinity preferably is used within a concentration range such that it easily permeates the structure of the biodegradable polymer, for example sponge, so that it can completely or partially cover the surface of the structure or can form a porous structure. If the concentration of the material is low, the structure of the biodegradable polymer cannot be covered or the porous structure cannot be formed. On the other hand, if the concentration is high, the viscosity of the solution of the material is too high, so the solution of the material does not fully penetrate the structure of the biodegradable polymer. For this reason, in the case of collagen, the concentration range for use is 0.01 to 15 wt%, and preferably 0.1 to 1.5 wt%. Additives such as a surfactant, astabilizer, an anti-oxidant, a pH adjusting agent and an antibacterial agent may further be added to the solution having physiological affinity as necessary.

After solubilization to give a liquid, the material having physiological affinity may be subjected to cross-linking treatment and thus to form cross-linkages between the materials and/or between the material and the biodegradable polymer. If cross-linking treatment of the material is performed, the mechanical strength increases, no defects occur when regenerated bone tissue or a bone treatment material is stapled to the body, and the bone tissue or the bone treatment material can be supported while maintaining the required strength until the bone tissue has fully regenerated. In this case, the structure of the biodegradable polymer (e.g., sponge, unwoven cloth, cotton) is impregnated with the solubilized material having physiological affinity, and cross-linkages are formed between molecules of the material and/or between the molecules of the material and the biodegradable polymer by a method known in the art (e.g., irradiation with ultraviolet rays or gamma rays, or by a chemical cross-linking technique using glutaraldehyde, vinyl sulfone or the like).

Examples of cross-linking agents which can be used to cross-link the material having physiological affinity include aldehydes (such as glutaraldehyde and formaldehyde), glycidyl ethers (such as ethylene propylene diglycidyl ether, glycerol polyglycidyl ether, diglyceryl polyglycidyl ether, sorbitol polyglycidyl ether and ethylene glycol diglycidyl ether), isocyanates (such as hexamethylene diisocyanate, α-tolidine isocyanate, tolylenediisocyanate, napthalene-1-5-diisocyanate, 4,4-diphenylmethane isocyanate, and triphenylmethane-4,4,4-triisocyanate), and calcium gluconate.

In an embodiment of this invention, the structure of the biodegradable polymer (e.g., sponge, unwoven cloth, cotton) is impregnated with the solubilized collagen solution, and is then cross-linked between the collagens and/or between the collagen and the biodegradable polymer having mechanical strength by a method known in the art. The collagen solution for use may be any solution known in the art, but enzyme-solubilized collagen solution is particularly preferred as teropeptides with immunogenicity are removed. A method of cross-linking collagen is also disclosed in, e.g. Stefan Nehrer et al., Tissue Eng. 4, pp.175-183 (1998).

In another embodiment of this invention, to introduce the porous structure of the material having physiological affinity into the sponge of the biodegradable polymer having mechanical strength, the biodegradable polymer sponge is immersed in the aqueous solution of the material having physiological affinity under reduced pressure, so that the aqueous solution fully permeates the pores of the biodegradable polymer sponge. Subsequently, after freezing, by lyophilizing this under vacuum, a sponge of the material having physiological affinity can be formed in the biodegradable polymer sponge having mechanical strength.

This invention will be explained in greater detail by way of the examples hereinbelow; however, the invention will not be restricted to those example.

### Examples

### (Example 1) Production of porous material

A porous material was produced using polylactic acid-polyglycolic acid copolymer (PLGA) (copolymer of lactic acid: glycolic acid = 75:25, 90-120 kDa) as the biodegradable polymer, and collagen Type I as the material having physiological affinity.

As the first step, a PLGA sponge was produced by particulate leaching. The PLGA polymer was dissolved in chloroform to prepare a PLGA polymer solution (concentration 20 (w/v) %) . Sodium chloride (9 g) having a particle diameter of about 355-425 µm was added to 5 ml of this solution and vortexed, and poured into an aluminum pan. Subsequently, chloroform was evaporated by air-drying in a draft for 24 hours, followed by drying under reduced pressure for 24 hours. The sodium chloride was removed by washing with deionized water, and a PLGA sponge with about 90% porosity resulted.

Next, the PLGA sponge was solubilized with protease under reduced pressure, and immersed in an acidic solution of bovine collagen Type I from which antigenicity had been removed (KOKEN Co., Ltd.) (pH 3.2, 5 µg/µL) so that the pores of the PLGA sponge were filled with collagen solution. This was then frozen at -80 °C for 12 hours, and lyophilized under a vacuum of 0.2 Torr for 24 hours so as to form a porous collagen structure in the PLGA sponge. The obtained material was cross-linked by treatment with the vapor of a 25% glutaraldehyde saturated aqueous solution at 37 °C for 4 hours; and unreacted aldehyde groups were blocked by treating with 0.1 M glycine aqueous solution. After washing this with deionized water, and lyophilizing again, a porous material comprising cross-linked collagen and PLGA sponge was obtained.

### (Example 2) Mesenchymal stem cells

A C3H/He mouse that was syngeneic to KUSA/A1 cells was anesthetized with ether, the femur was excised, and mesenchymal stem cells were extracted from the bone marrow (Dexter, T.M. et al., J. Cell Physiol. 91 (1977), pp.335-344) . Subsequently, the extracted cells were cultured in an IMDM medium (Sigma Co.) supplemented with 20% FBS and penicillin (100 µ g/ml)/streptomycin (250 ng/ml) under 5% CO₂. Monolayer cells obtained from repeated subcultures (KUSA/A1 cells) (deposited on July 11, 2001 to the National Institute of Advanced Industrial Science and Technology, International Patent Organism Depositary (Tsukuba Central 6, 1-1-1, Higashi, Tsukuba, Ibaraki, Japan,) with Accession No. FERM P-18414, and transferred on August 19, 2002 to deposition under the Budapest Treaty with Accession No. FERM BP-8156) were maintained in an IMDM medium supplemented with 10% FBS and penicillin (100 µg/ml)/streptomycin (250 ng/ml) .

Next, the DNA content, proliferation ability (DNA content), alkaline phosphatase activity, Ca content and osteocalcin (BGP) production were examined for the obtained cells (KUSA/A1 cells) and fibroblasts (MC3T3-E1 cells) for reference.

### (1) Measurement of DNA content

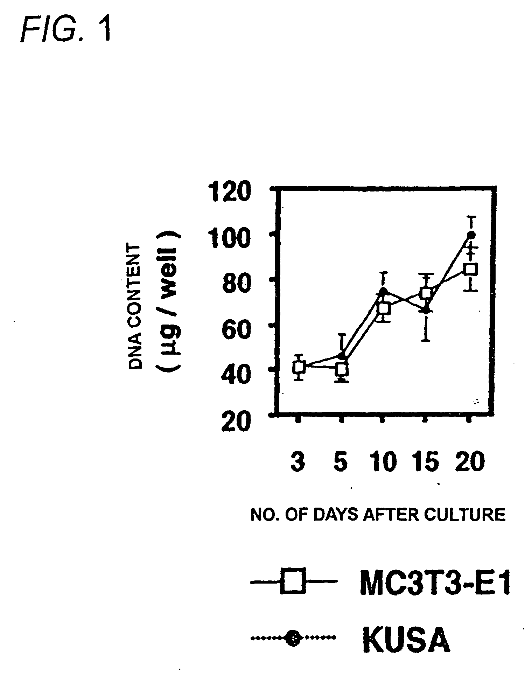

Cells were inoculated on a 24-well plate, and grown in an IMDM medium supplemented with 10% FBS and penicillin (100 µg/ml) /streptomycin (250 ng/ml). Subsequently, the cells were homogenized in a homogenization buffer (20 mM Tris-HCl, pH 7.2, 0.1% Triton X-100). Hoechst 33258 buffer (1 µg/ml Hoechst 33258, 0.05 M Na₃PO₄, 2.0 M NaCl, 2.0 mM EDTA, pH 7.41, Hoechst Marion Roussel Corp., Cincinnati, Ohio, USA) was mixed to this sample (50 µl). The fluorescence was measured by a spectrophotometer at an excitation wavelength of 356 nm and at a wavelength of 458 nm. As a result, the amount of DNA increased with time in both the KUSA/A1 cells and MC3T3-E1 cells, indicating that both cells proliferated normally (FIG. 1).

### (2) Measurement of alkaline phosphatase (ALP) activity

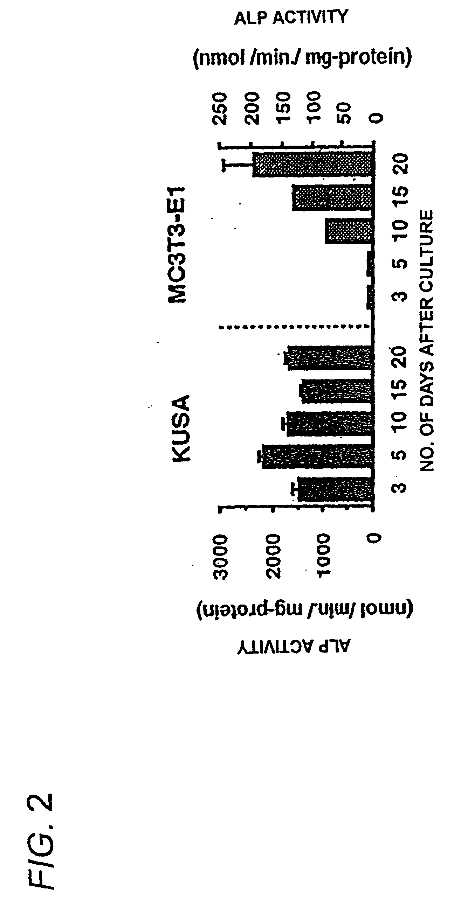

Cells cultured similarly to (1) were washed twice with PBS, transferred to 1 ml of 0.9% NaCl, and homogenized on ice. Subsequently, the ALP activity of each sample was measured on the spectrophotometer at a wavelength of 405 nm using anALP B-test Waco kit (Wako Pure Chemical Industries, Ltd.). As a result, for the KUSA/A1 cells, ALP activity which is known to be high for bone tissue was about 100 times higher than that for MC3T3-E1 cells, indicating that the KUSA/A1 cells have the character of osteoblasts (Fig. 2).

### (3) Ca content and osteocalcin (BGP) production

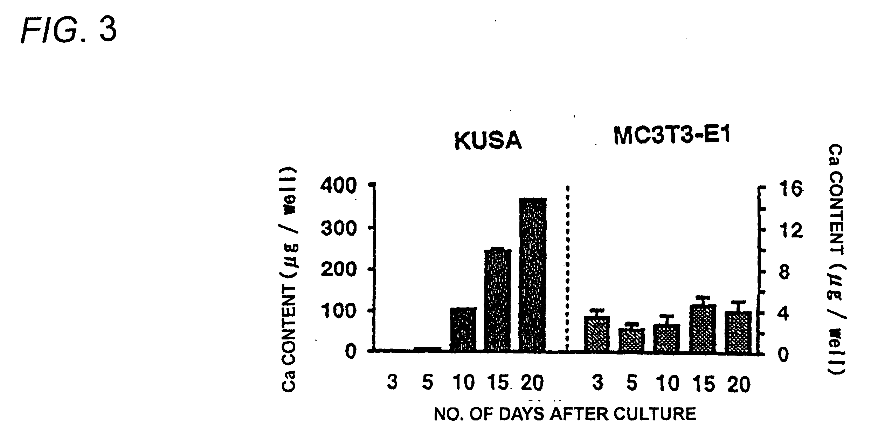

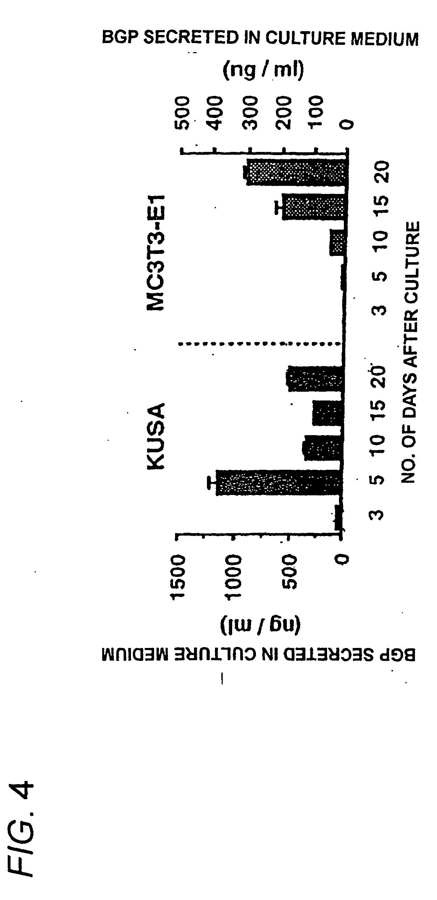

Cells cultured similarly to (1) were washed twice with PBS, and sonicated in homogenization buffer (20mM Tris-HCl, pH 7.2, 0.1% Triton X-100). Here, the Ca content of the obtained sample was measured by a Calcium C test (Wako Pure Chemical Industries, Ltd.). Osteocalcin production was measured by RIA using amouse osteocalcin assay kit (Biomedical Technologies Inc. , Stoughton, MA) . As a result, for the KUSA/A1 cells, the Ca content (Fig. 3) and osteocalcin production (Fig. 4) were notably higher than those of MC3T3-E1 cells, again indicating that the KUSA/A1 cells have the character of osteoblasts.

### (Example 3) Bone regeneration in formedporous material

KUSA/A1 cells (1x10⁷/ml) were suspended in 1 ml of culture liquid, and this suspension was applied in layers 5 times over several days (sum total of number of cells: 5x10⁷) to a cube (approx. 0.8x0.8x0.8 cm) of porous material comprising the collagen and PLGA sponge produced in Example 1. Next, this was implanted to the subcutaneous tissue and the abdominal cavity of a mouse. The implanted cube was observed by X-rays at 6 and 11 weeks after implantation. It was then removed, and bone regeneration was confirmed histologically.

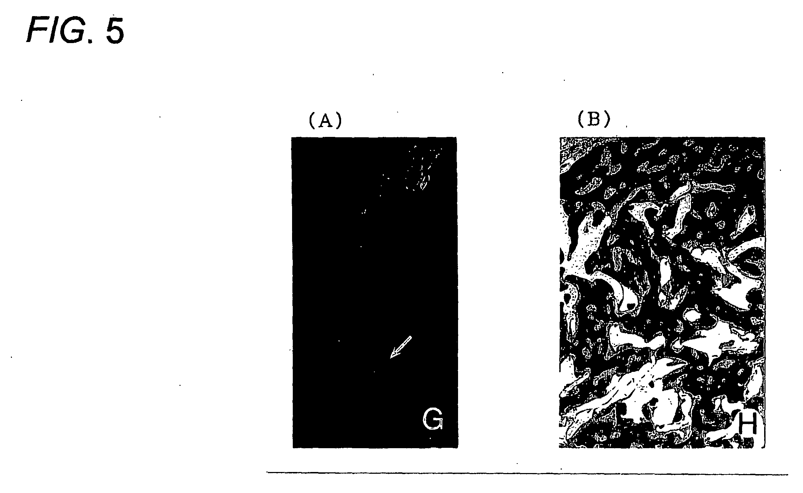

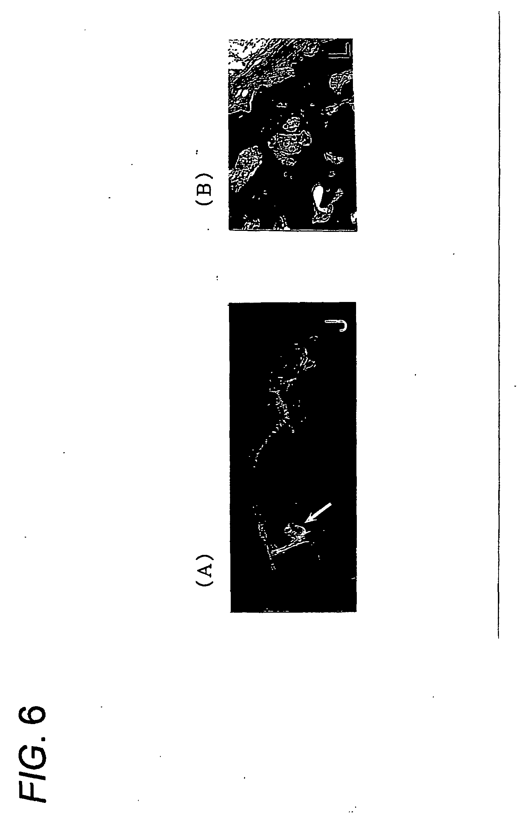

The results were that bone regeneration in the porous material was noted by X-rays and histological observations after 6 weeks (Fig. 5). In Fig. 5, A is a soft-X-ray photograph of the regenerated bone (6 weeks after implantation) , and it is seen that regenerated bone having the same shape as the porous material (arrow) was formed 6 weeks after implanting the porous material to the mouse abdominal cavity. B is a tissue image (hematoxylin/eosin staining) of the regenerated bone, and regenerated bone is clearly seen. At 6 weeks, porous material remains, and this appears as an unstained image (white image) which has escaped staining. After 11 weeks, the porous material was replaced by bone tissue, and the bone tissue was completely regenerated (Fig. 6). In Fig. 6, A is a soft-X-ray photograph of the regenerated bone (6 weeks after implantation), and completely regenerated bone having the same shape as the porous material (arrow) was formed at 11 weeks after implanting the porous material to the mouse abdominal cavity. B is a tissue image (hematoxylin/eosin staining) of the regenerated bone, and perfectly regenerated bone accompanied by dense trabecula was observed. At 11 weeks, the porous material had been absorbed.

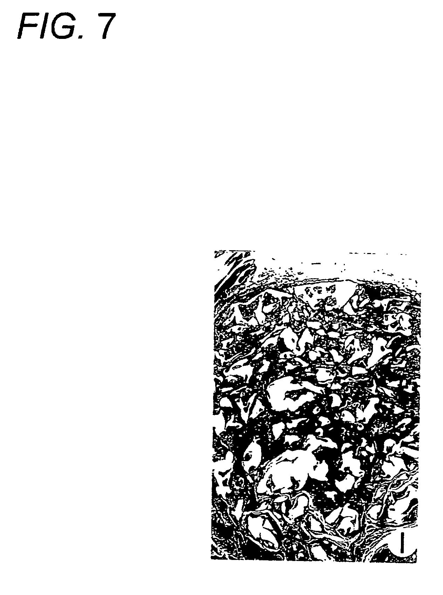

On the other hand, when only a porous material comprising collagen and PLGA sponge was implanted, bone regeneration was not observed at all (Fig. 7). Fig. 7 is its tissue image. There was no osteogenesis at all, and reaction to foreign bodies was observed inside and around the porous material.

### (Example 4) Implantation of KUSA/A1 cell-inoculated material to a flat bone defect mouse

A Vicryl^{R} knitmesh (PLGAmesh, Johnson & Johnson, Inc. ) , a copolymer obtained by polymerizing commercial glycolic acid and lactic acid in a proportion of 9:1, was immersed in an acidic aqueous solution of bovine collagen Type I (pH = 3.2, 5 µg/m µl), and frozen to -80 °C for 12 hours. Subsequently, this frozen material was lyophilized under vacuum (0.2 Torr) for 24 hours, and after carrying out cross-linking treatment for 4 hours with saturated glutaraldehyde vapor from 25 wt% glutaraldehyde aqueous solution at 37 °C, it was immersed in 0.1 M glycine aqueous solution for 4 hours. This was washed with deionized water, then lyophilized again, and a fibrous material comprising cross-linked collagen and a PLGA mesh was thus prepared.

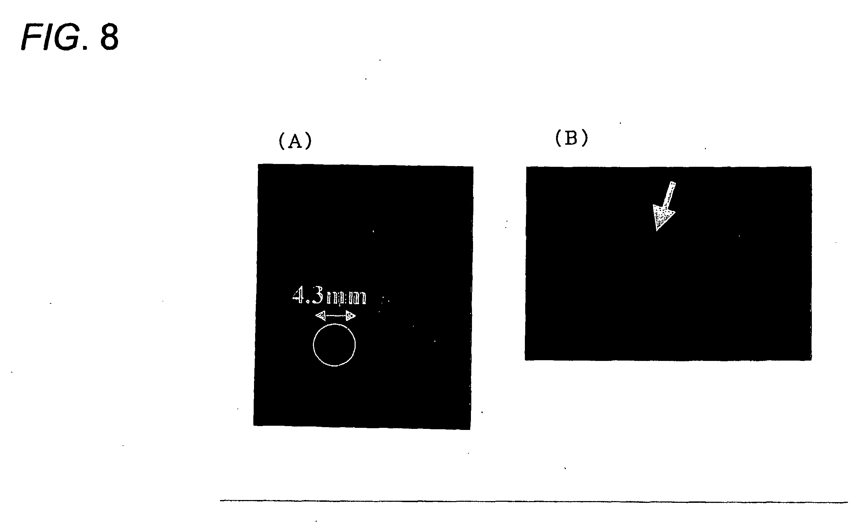

Next, KUSA/A1 cells were inoculated on the fibrous material comprising the cross-linked collagen and PLGA mesh at 1x10⁷/ml. and cultured in an incubator for 1 week. The skull of a C3H/He mouse was exposed under anesthesia, the bone was excavated with a circular drill of diameter 4.3 mm, and a bone defect was created (Fig. 8(A)). The cell-inoculated fibrous material was placed on the defect so as to cover it, the skin was sutured, and rearing of the mouse was continued. After 4 weeks, the skull was extracted from the mouse, and observation byX-rays was performed. As a control, a bone defect mouse wherein only fibrous material was placed without cell inoculation, was produced.

When cell inoculation was not performed, there was only granulation in the defect, and bone regeneration was not observed. By contrast, when the inoculation of KUSA/A1 cells was performed, osteogenesis proceeded well and the defect was completely covered by bone (Fig. 8(B)).

### (Example 5) Implantation of KUSA/A1 cell-inoculated material to a long tube bone defect mouse

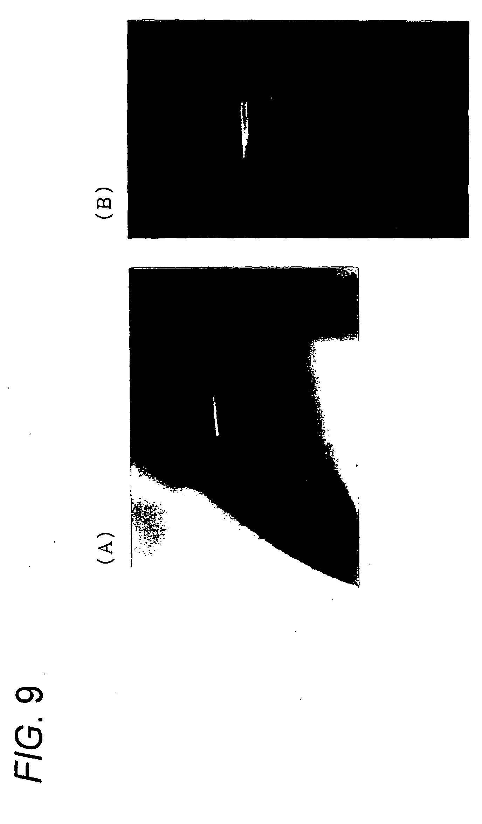

A fibrous material inoculated with KUSA/A1 cells was cultured in an incubator for 1 week similarly to Example 4. The femur of a C3H/He mouse was exposed, a bone defect was created in the center of the diaphysis with a 4.3-mm drill, and a stainless steel rod was installed in the center of the cavum medullare in order to compensate the instability due to the bone defect (Fig. 9(A)). The circumference of the defect was covered with the cell-inoculated fibrous material. After rearing for 4 weeks, X-rays and histological observations of the femur were performed.

Satisfactory osteogenesis of the implant was confirmed by X-rays (Fig. 9(B)). Histologically, neither inflammation nor strong foreign body reaction was observed around the fibrous material. At 4 weeks after implantation, union with the normal bone stump could not be confirmed.

### Industrial Applicability

According to this invention, by allowing mesenchymal stem cells or osteoblasts having the multi-potentiality for differentiation to proliferate in a fibrous and/or porous material which can serve as a scaffold for these cells, a method for rapidly generating and regenerating bone tissue having a specific shape and size, and which is suitable for implantation, and a regenerated bone treatment material obtained by this method, are provided. This method is suited for regeneration of bone tissue for autologous implantation and heterologous implantation, and the obtained regenerated bone treatment material is free from concern of rejection and finds usefulness.

Therefore, the method and the regenerated bone treatment material of this invention permit the therapy of diseases related to bone loss, bone fracture and bone adhesion.

## Claims

1. A method of regenerating bone, comprising allowing mesenchymal stem cells to proliferate in a fibrous and/or porous material which can serve as a scaffold for the cells.

2. The method according to claim 1, wherein the material comprises a combination of a material having physiological affinity and a biodegradable polymer having mechanical strength.

3. The method according to claim 1 or 2, wherein the material having physiological affinity is one or more biomaterials selected from the group consisting of collagen, polyethylene oxide, fibrin glue, gelatin, fibronectin, laminine, proteoglycan and glycosaminoglycan.

4. The method according to claim 1 or 2, wherein the biodegradable polymer having mechanical strength is one or more biodegradable polymers selected from the group consisting of polyglycolic acid, polylactic acid, polylactic acid-polyglycolic acid copolymer, polymalic acid, chitin, chitosan, polyalginic acid, hyaluronic acid, cellulose, polyamino acid and an ω-hydroxycarboxylic acid.

5. The method according to any of claims 1-4, comprising using cells isolated from a living body or cultured cells thereof.

6. The method according to any of claims 1-5, wherein the material comprising a combination of a material having physiological affinity and a biodegradable polymer having mechanical strength is shaped, and the regeneratedbone of desired shape and size is obtained by allowing the cells to proliferate in the shaped material.

7. A regenerated bone tissue obtained by the method according to any of claims 1-6.

8. A bone treatment material produced by the method according to any of claims 1-6.
